Europäisches Patentamt

European Patent Office

Office européen des brevets

(19)

(11) **EP 0 471 174 B1**

(12) **EUROPÄISCHE PATENTSCHRIFT**

(45) Veröffentlichungstag und Bekanntmachung des Hinweises auf die Patenterteilung:
**20.03.1996 Patentblatt 1996/12**

(51) Int Cl.6: **G01N 1/22**, G01N 33/00, G01M 15/00

(21) Anmeldenummer: **91110835.5**

(22) Anmeldetag: **29.06.1991**

(54) **Anlage zur Schadstoffanalyse, insbesondere Partikelemission von Dieselmotorenabgas, mit einer speziellen Teilstromverdünnungseinrichtung**

Installation for analyzing pollutants, in particular particulate emission of exhaust gas of diesel engines, with a dilution device for a partial current

Installation pour l'analyse d'éléments polluants, en particulier d'émissions de particules de gaz d'échappement de moteurs diesel, avec un dispositif de dilution d'un courant partiel

(84) Benannte Vertragsstaaten:
**CH DE FR GB IT LI NL SE**

(30) Priorität: **14.08.1990 AT 1690/90**

(43) Veröffentlichungstag der Anmeldung:
**19.02.1992 Patentblatt 1992/08**

(73) Patentinhaber: **STEYR NUTZFAHRZEUGE AG**
**A-4400 Steyr (AT)**

(72) Erfinder:
- **Schlögl, Harald, Dr. Dipl.-Ing.**
  **A-4400 Steyr (AT)**
- **Richter, Klaus**
  **A-4523 Neuzeug (AT)**

(56) Entgegenhaltungen:
| | |
|---|---|
| EP-A- 0 155 793 | EP-A- 0 316 688 |
| EP-A- 0 388 392 | EP-A- 0 428 850 |
| US-A- 3 603 155 | |

- STAUB-REINHALTUNG DER LUFT Bd. 46, Nr. 2, Februar 1986, Düsseldorf, DE, SS. 56-59; T. GAST et al.: 'Gesichtspunkte zur Teilstromentnahme aus Abgaskanälen'

**Beschreibung**

Die Erfindung betrifft eine Anlage zur Schadstoffanalyse, insbesondere Partikelemission, von Dieselmotorenabgas, mit einer Teilstromverdünnungseinrichtung mit Mitteln zur Einstellung und Bestimmung des Abgas-/Luft-Verdünnungsverhältnisses und mit einer Abgasaufnahmeleitung, der die Abgase eines zu testenden Motors zuführbar sind.

Die Erfindung geht aus von Forschungs- und Testergebnissen, über die im Artikel "Zur Partikelemission von Nutzfahrzeug-Dieselmotoren", Seiten 186 bis 193 der MTZ Motortechnische Zeitschrift 51 (1990) 5, Mai 1990 berichtet wird. Dabei wird auch Bezug genommen auf Anlagen zur Schadstoffanalyse von Dieselmotorabgas und dabei insbesondere der Partikelemission. Im Rahmen solcher Anlagen kamen bisher Teilstromverdünnungsverfahren zur Anwendung, die sich im wesentlichen an den EPA (Environment Protection Agency)-Vorschriften der USA orientierten. Die Einstellung und Bestimmung des Abgasteilungsverhältnisses und damit des Abgas-/Luft-Verdünnungsverhältnisses erfolgten bei diesem bekannten Verfahren unter Verwendung einer Nulldrucksonde (IKTM-Sonde).

Die Teilstromverdünnungseihrichtung weist in diesem Fall einen Mischkanal, dem ein Abgasteilstrom und Luft zugeführt wird, sowie zwei Gebläse - ein Druckgebläse zur Einblasung von Verdünnungsluft und ein Sauggebläse zur Absaugung des verdünnten Abgas-Luft-Gemisches - auf. Daraus ergab sich eine relativ voluminöse Gesamtanordnung mit außerdem erheblichen Kosten für die Gebläse. Außerdem muß bei diesem bekannten Verfahren die Drehzahl des Sauggebläses in Abhängigkeit der Differenz der in der Abgasentnahmesonde und im Abgasrohr gegebenen statischen Drücke geregelt werden, was einen erheblichen regelungs- und steuerungstechnischen Aufwand erfordert. Die notwendige Nulldrucksonde reagiert sehr empfindlich auf Druckschwankungen, so daß für deren Anordnung, um exakte I..iessungen zu bekommen, aufwendige Dämpfungsmittel notwendig sind. Die Abgasentnahmemenge ist zudem durch die Sondengeometrie vorgegeben, mit dem Nachtteil, daß die Aufwendbarkeit der solchermaßen bestückten Anlage im Hinblick auf den unterschiedlich hohen Abgasausstoß der zu testenden Motoren über den gesamten Lastbereich zu wenig variabel ist.

Es ist daher Aufgabe der Erfindung, für eine Anlage der eingangs definierten Art eine Teilstromverdünnungseinrichtung zu schaffen, die weniger Bauraum und Kosten für ihre Realisierung beansprucht und es außerdem ermöglicht, das Abgas-/Luft-Verdünnungsverhältnis derart einzustellen, daß hierauf basierend eine exakte Schadstoffanalyse, insbesondere der Partikelemission, des über dem gesamten Lastbereich zu testender, auch unterschiedlich hubraumgrößer Motoren ausgestoßenen Abgases durchführbar ist.

Diese Aufgabe ist erfindungsgemäß durch eine Teilstromverdünnungseinrichtung gemäß den Merkmalen des Anspruchs 1 gelöst.

Vorteilhafte Ausgestaltungen dieser Lösung sind in den Unteransprüchen angegeben.

Im einzelnen weist die erfindungsgemäße Teilstromverdünnungseinrichtung die Folgenden Merkmale auf:

- eine Abgasentnahmesonde, mit der Motorabgas aus der Abgasaufnahmeleitung entnehmbar und über eine diesen Massenstrom $\dot{m}_{SON}$ messende Massenstromsonde sowie eine anschließliche Abgaseinspeiseleitung in einen Mischkanal einspeisbar ist, der einenendes an einem eingangs eine Einlaufdüse mit Wirkdruckmeßeinrichtung aufweisenden Verdünnungsluftansaugkanal und anderenendes über einen Absaugkanal an ein die luftverdünnten Abgase absaugendes Gebläse angeschlossen ist und aus dessen Mischzone luftverdünnte Abgase für Analysezwecke über entsprechende Entnahmeorgane abführbar sind,

- je eine Drosselklappe vor bzw. hinter dem Mischkanal in den dortigen Leitungswegen zur Einstellung der jeweiligen Massenströme und Drücke,

- je eine Drosselklappe strömungsmäßig vor bzw. hinter der Abgasentnahmestelle in der Abgasaufnahmeleitung für eine einem störungsfreien Betrieb des angeschlossenen Motors bewirkende Abgasgegendurckeinstellung und eine Abgasdruckvariierung im Abgasentnahmebereich, sowie

- Regel- und Steuereinrichtungen zur bedarfsgerechten Einstellung des Abgas-/Luft-Verdünnungsverhältnisses

$$q=\frac{\dot{m}_{DIL} + \dot{m}_{SON}}{\dot{m}_{SON}}$$

durch entsprechende Drosselklappenverstellung anhand erfaßter Massenstromwerte $\dot{m}_{DIL}$ (Verdünnungsluftstrom, gemessen an Einlaufdüse), $\dot{m}_{SON}$ (Abgasteilstrom, gemessen mit Massenstromsonde).

Im Vergleich zu der bekannten, bisher verwendeten Anlage benötigt die erfindungsgemäße Teilstromverdünnungseinrichtung nur mehr ein Gebläse, wodurch sich gebläseseitig die Kosten etwa halbieren und weniger Baumraum benötigt wird. Außerdem ist eine vergleichsweise einfache und kostengünstige Messung der Verdünnungsluft möglich. Es entfällt außerdem die komplizierte Drehzahlregelung des Sauggebläses. Ferner ist keine aufwendige Dämpfungseinrichtung im Bereich der Abgasentnahmesonde mehr notwendig, weil sich die auftretenden Druckpulsationen nicht negativ an der erfindungsgemäßen Abgasentnahmeanordnung auswirken können. Ferner können vorteilhafterweise die Abgase unterschiedlich großer Motoren, die über deren gesamten Lastbereich anfallen, exakt analysiert werden.

Das geringe Baumvolumen ermöglicht schließlich die Ausbildung der Gesamtanlage als mobiles System.

Nachstebend ist die erfindungsgemäße Lösung anhand der Zeichnung noch näher erläutert. In der Zeichnung zeigen:

Fig. 1      eine Schemadarstellung der Gesamtanlage zur Schadstoffanalyse,

Fig. 2      jenen Teil der Gesamtanlage gemäß Fig. 1 betreffend die Teilstromverdünnungseinrichtung,

Fig. 3 und 4      je eine Ansicht einer konkreten Ausführungsform der Gesamtanlage als mobiles Analysesystem.

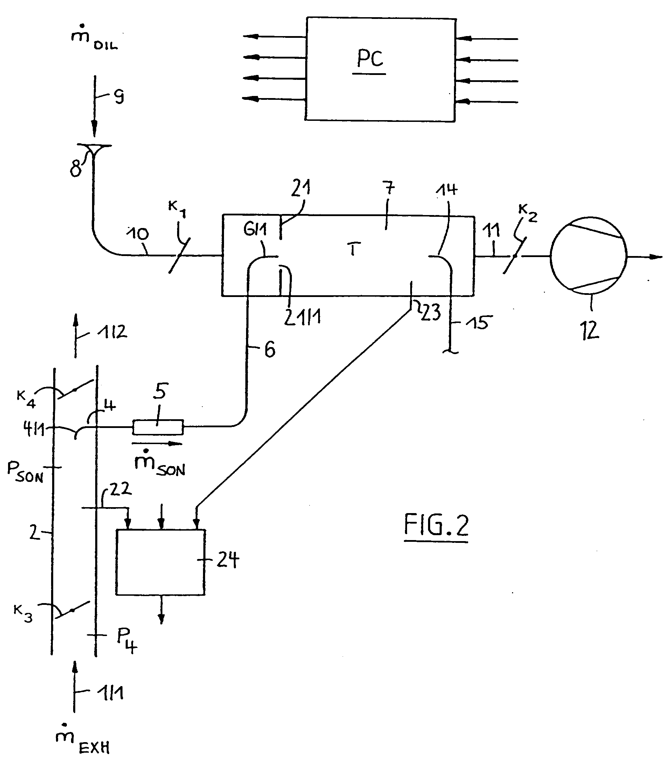

Die Anlage umfaßt eine Teilstromverdünnungseinrichtung gemäß Fig. 2 mit Mitteln zur Einstellung und Bestimmung des Abgas-/Luft-Verdünnungsverhältnisses sowie Einrichtungen zur Schadstoffanalyse, insbesondere der Partikelemission.

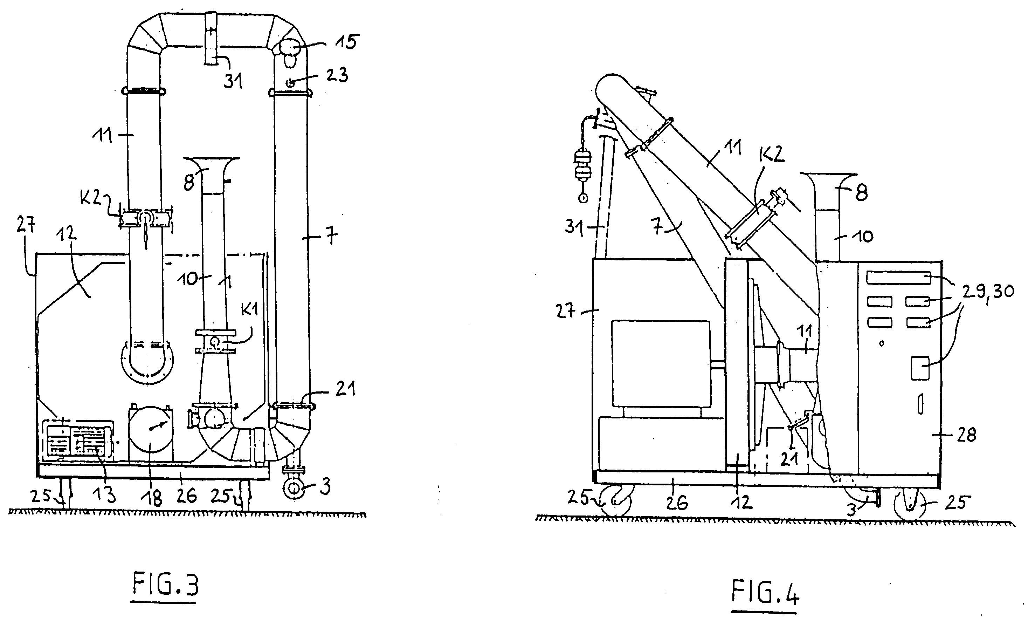

Der Abgasmassenstrom $\dot{m}_{EXH}$ eines zu testenden Dieselmotors wird in Richtung der Pfeile 1/1, 1/2 in eine anlageninterne Abgasaufnahmeleitung 2 ein- und durch diese hindurchgeleitet. Hierfür wird der Abgasstrang des betreffenden Dieselmotors über einen Verbindungsschlauch an einem aus Fig. 3 und 4 ersichtlichen Anschlußstutzen 3 angeschlossen. Mit einer Abgasentnahmesonde 4 wird ein Teilstrom $\dot{m}_{SON}$ des Motorabgases aus der Abgasentnahmeleitung 2 entnommen und über eine diesen Teilstrom $\dot{m}_{SON}$ messende Massenstromsonde 5 sowie eine anschließende Abgaseinspeiseleitung 6 in einen Mischkanal 7 eingespeist. Letzterer ist einenendes an einem eingangs eine Einlaufdüse 8 mit Wirkdruckmeßeinrichtung aufweisenden Verdünnungsluftansaugkanal 10, über den der Verdünnungsluftmassenstrom $\dot{m}_{DIL}$ in Richtung des Pfeiles 9 angesaugt wird, und anderenendes über einen Absaugkanal 11 an einem die luftverdünnten Abgase absaugenden Gebläse 12 angeschlossen.

Zur Einstellung der jeweiligen Durchflüsse und Drücke der Massenströme $\dot{m}_{EXH}$, $\dot{m}_{SON}$, $\dot{m}_{DIL}$ sind zwei Drosselklappen-Paare K1, K2 bzw. K3, K4 vorgesehen. Die in der Abgasaufnahmeleitung 2 strömungsmäßig vor der Abgasentnahmestelle gegebene Drosselklappe K3 dient zur Einstellung eines einen störungsfreien Betrieb des angeschlossenen Motors bewirkenden Abgasgegendruck $P_4$, während die strömungsmäßig hinter der Abgasentnahmestelle in der Abgasaufnahmeleitung 2 gegebene Drosselklappe K4 zur Variierung des Abgasdruckes $P_{SON}$ im Abgasentnahmebereich dient. Die Drosselklappe K2 im Absaugkanal 11 zwischen Mischkanal 7 und Gebläse 12 dient zur groben Einstellung des Abgas-/Luft-Massenstromes sowie zur gleichzeitigen Beeinflussung des Druckniveaus im Mischkanal 7. Die eigentliche Regelung und Einstellung des Abgas-/Luft-Verdünnungsverhältnisses erfolgt mittels der Drosselklappe K1. Durch eine zunehmende Drosselung des Verdünnungsluftmassenstromes $\dot{m}_{DIL}$ mit Drosselklappe K1 kann die in den Mischkanal 7 einströmende Verdünnungsluftmenge verringert werden. Gleichzeitig wird dabei das Druckgefälle zwischen der in der Abgasaufnahmeleitung 2 gegebenen Abgasentnahmestelle und dem Mischkanal 7 erhöht, mit der Folge, daß sich der in den Mischkanal 7 einströmende Abgasteilmassenstrom $\dot{m}_{SON}$ vergrößert.

Darüber hinaus verfügt die Anlage über Regel- und Steuereinrichtungen zur bedarfsgerechten Einstellung des Abgas-/-Luft-Verdünnungsverhältnisses

$$q = \frac{\dot{m}_{DIL} + \dot{m}_{SON}}{\dot{m}_{SON}}$$

durch entsprechende Verstellung der Drosselklappen K1, K2, K3, K4 anhand erfaßter Massenstromwerte $\dot{m}_{DIL}$ (gemessen an Einlaufdüse 8 mit Wirkdruckmeßeinrichtung) und $\dot{m}_{SON}$ (gemessen mit Massenstromsonde 5).

Aus der angegebenen Defination des Abgas-/Luft-Verdünnungsverhältnisses q ergibt sich die Möglichkeit einer Absenkung desselben und infolge davon eine Erhöhung der Temperatur T des luftverdünnten Abgases. Eine Erhöhung des Abgas-/Luft-Verdünnungsverhältnisses q und infolgedessen eine niedrigere Temperatur T des luftverdünnten Abgases ist durch eine geringere Drosselung des Verdünnungsluftmassenstromes $\dot{m}_{DIL}$ mittels der Drosselklappe K1 erzielbar. Auf diese Weise ist es somit möglich, für jeden Motorlastbetriebspunkt des zu testenden Motors das Abgas-/Luft-Verdünnungsverhältnis q so einzustellen, daß die Temperatur T des luftverdünnten Abgases weitgehend konstant auf einen Wert T ≤ 51,7°C haltbar ist, was z. B. eine Forderung der EPA (Environment Protection Angency)-Vorschriften der USA für solche Abgasanalyseverfahren ist.

Das solchermaßen temperaturstabilisierte luftverdünte Abgas wird für eine Partikelanteilsanalyse aus der Mischzone des Mischkanals 7 mittels einer Vakuumpumpe 13 über ein entsprechendes Entnahmeorgan 14, z. B. eine Partikelsonde, und eine anschließende Saugleitung 15 abgesaugt und - während einer Sammelperiode über zwei Partikelfilter 16, 17, außerhalb einer Sammelperiode über eine Bypaßeinrichtung an letzteren vorbei - nachgeordneten Auswerteinrichtungen zugeführt. Bei letzteren handelt es sich um einen der Vakuumpumpe 13 nachgeschalteten Gaszähler 18, der mit einem Impulszähler ausgestattet ist. Die Bypaßleitung dient dazu, eine kontinuierliche Gasentnahme aus dem Mischkanal 7 zu ermöglichen, auch wenn keine Sammelperiode vorgesehen ist. Die Bypaßeinrichtung besteht beispielsweise aus einem elektropneumatisch geschalteten Ventil 19 in der Saugleitung 15 und einer hiervon abzweigenden sowie nach den Partikelfiltern 16, 17 wieder in sie einmündenden Bypaßleitung 20.

Beginnend mit einer Umschaltung des Ventils 19 in Stellung für Sammelbetrieb, erfolgt durch den Gaszähler 18 eine Aufsummierung der von den Partikeln ausgelösten Impulse; außerdem wird die Sammelzeit mitgestoppt. Auf weitere Einzelheiten der Auswerteinrichtungen und des Analyseverfahrens sei nicht eingegangen, da dies nicht Gegenstand der Erfindung ist.

Nachstehend ist auf verschiedene Details der Teilstromverdünnungseinrichtung näher eingegangen.

Das rohrförmige Gasaufnahmeteil 4/1 der Abgasentnahmesonde 4 ist mittig, insbesondere koaxial in der Abgasaufnahmeleitung 2 verlaufend im Rohabgasstrom angeordnet und anschließend abgewinkelt nach außen zur Massenstromsonde 5 hingeführt. Ferner ist der Abgasaufnahmeteil 4/1 der Abgasentnahmesonde 4 räumlich, vorzugsweise kurz vor der Drosselklappe K4 angeordnet, weil in diesem Bereich zwischen den Drosselklappen K3, K4 hinsichtlich Druck und Strömung die an sich günstigsten Abgasentnahmeverhältnisse gegeben sind. Die sich an der Massenstromsonde 5 anschließende und seitlich in den Mischkanal 7 hineingeführte Abgaseinspeiseleitung 6 ist mit ihrem austrittsbereichsseitigen Ende 6/1 mittig und insbesondere konzentrisch im Mischkanal 7 situiert und endet dort mit ihrer Austrittsöffnung vor oder im Bereich der Durchlaßöffnung 21/1 einer Lochblende 21, nach der die Abgas-/Luft-Mischstrecke beginnt und die die Vermischung der beiden Teilströme Abgas/Luft begünstigt.

Bei der in Fig. 3 und 4 dargestellten konkreten Ausführungsform hat die Abgas-/Luft-Mischstrecke bei einem Mischkanalrohrinnendurchmesser von ca. 150 mm eine Länge von etwa 1450 mm. Der Durchmesser der Durchlaßöffnung 21/1 in der Lochblende 21 beträgt etwa 100 mm und der Mündungsteil 6/1 der Abgasspeiseleitung 6 hat einen Durchmesser von etwa 25 mm. Der Abgasentnahmeteil 4/1 der Abgasentnahmesonde 4 hat bei einem Durchmesser der Abgasaufnahmerohrleitung 2 von ca. 100 mm dagegen nur einen Durchmesser von etwa 12 mm.

Das einzig notwendige Gebläse 12 ist ein vorzugsweise mit konstanter Drehzahl laufendes Saugebläse.

Der durch eine Rohrverbindung gebildete Verdünnungsluftansaugkanal 10 hat im Bereich nach der Einlaufdüse 8 einen etwa 2/3 jenes des Mischkanals 7 betragenden Durchmesser und erweitert sich im Bereich nach der Drosselklappe K1 kontinuierlich auf den Durchmesser des den Mischkanal 7 bildenden Rohres. Die Einlaufdüse 8 ist durch ein Venturirohrstück gebildet, das mit seinem verengten Trichterquerschnitt am Ende des Verdünnungsluftansaugkanales 10 austauschbar angeschlossen ist. Sogesehen besteht die Möglichkeit, zum Analysieren der Abgase unterschiedlich hubraumgroßer Motoren entsprechend angepaßte, unterschiedlich groß kalibrierte Einlaufdüsen 8 verwenden zu können.

Aufgrund dieser Anordnung wird somit die Verdünnungsluft aus der Atmosphäre, in der Regel aus der Umgebung der Anlage entnommen und ungefiltert sowie ohne über einen Wärmetauscher geführt zu werden durch den Verdünnungsluftansaugkanal 10 in den Mischkanal 7 eingespeist.

Die Einlaufdüse 8 ist mit einem nicht gezeigten Druckwandler kombiniert, der den Wirkdruck der angesaugten Verdünnungsluft erfaßt, kleine Über-, Unter- und Differenzdrücke feststellt und einerseits ein druckproportionales Spannungssignal zur Weiterverarbeitung liefert, andererseits die erfaßten Werte auch auf einer Anzeigeeinrichtung, vorzugsweise einem LCD-Display optisch zur Anzeige bringt.

Mit der Massenstromsonde 5 wird der von der Abgasentnahmesonde 4 aufgenommene Abgasteilstrom $\dot{m}_{SON}$ über eine Durchflußmessung nach dem Staudruckprinzip, resultierend aus den Druck- und Strömungsgegebenheiten in der Abgasaufnahmeleitung 2, bestimmt.

Dieses Meßprinzip basiert auf einer Geschwindigkeitsmessung, bei der der Differenzdruck zwischen statischem Druck und Gesamtdruck (Staudruck) gemessen und über die Bernoulligleichung die Strömungsgeschwindigkeit bestimmt wird. Weiters wird die Dichte des Abgasteilstromes $\dot{m}_{SON}$ nach dem Annubarprinzip berechnet, um auf den konkreten Wert des Massenstromes $\dot{m}_{SON}$ zu kommen. Die Messung des Differenzdruckes erfolgt mit einem speziellen, im Handel erhältliche Differenzdrucktransmitter, z. B. Rosemount-Typ 1151-DR2-F22-C1-B2-M1-L4, oder mittels eines Schrägrohrmanometers mit Ethanolfüllung, dessen Auflösung 0,4 nm WS (Genauigkeit < 1%) beträgt.

Zur Erfassung und Verarbeitung dieser und anderer Meßwerte dient ein anlageneigenes Computersystem PC, das hard- und softwaremäßig zweckentsprechend ausgestattet ist und unter anderem zumindest teilweise, vorzugsweise jedoch vollständig die Regelung und Steuerung des Abgas-/Luft-Verdünnungsprozesses durch entsprechende Befehle an die Stellorgane der Drosselklappen K1, K2, K3, K4 übernimmt.

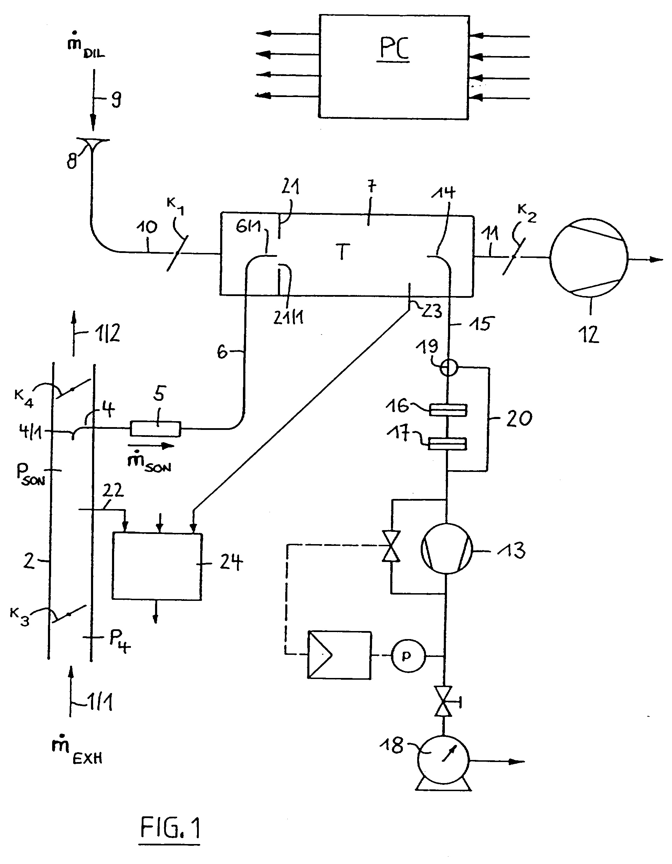

Zusätzlich zu den vorgenannten Einrichtungen, die die Bestimmung des Abgas-/Luft-Verdünnungsverhältnisses q nach der Massenbilanz-Methode ermöglichen, können - wie in Fig. 1 und 2 dargestellt - Einrichtungen zur Messung des $CO_2$-Gehaltes des Rohabgases (Erfassungsorgan 22), der Verdünnungsluft (nicht dargestellt) und des verdünnten Abgases (Erfassungsorgan 23) vorgesehen sein, welche Meßwerte einem $CO_2$-Analysator 24 zugeführt werden. Mit diesem ist eine Bestimmung des Abgas-/Luft-Verdünnungsverhältnisses nach der $CO_2$-Bilanzmethode durchführbar, deren Ergebnisse zu Vergleichszwecken mit den Ergebnissen der normalerweise durchgeführten Massenbilanz-Methode heranziehbar sind. Die diesbezügliche Auswertung erfolgt im Computersystem PC.

Wie aus Fig. 3 und 4 ersichtlich, kann die gesamte Anlage als mobile Einheit konzipiert sein, wozu alle Teile derselben auf bzw. an einer mit Rädern 20 versehenen Plattform 26 angeordnet sind, die beliebig verfahrbar ist. An dieser Plattform 26 sind die einzelnen Aggregate, teilweise auch die genannten, Mediumströme führenden Leitungen innerhalb einer äußeren Verschalung/Verkleidung 27 (teilweise aufgebrochen dargestellt) untergebracht. Ein als Wandteil oder schwenkbare Tür ausgebildeter Teil 28 der Außen-Verschalung 27 ist mit verschiedenen Anzeigen/Anzeigeinstrumenten 29 und Bedienungsorganen 30, wie Schalter, Tastaturen und dergleichen ausgestattet, welche mit dem internen Computersystem PC und/oder anderen Teilen der Anlage elektrisch über entsprechende Verbindungsleitungen verbunden sind. Mit 31 ist schließlich noch eine Stütze bezeichnet, die die Lage der medienführenden Rohre 10, 7, 11 stabilisiert.

**Patentansprüche**

1. Anlage zur Schadstoffanalyse, insbesondere Partikelemission, von Dieselmotorenabgas, mit einer Teilstromverdünnungseinrichtung zur Einstellung und Bestimmung des Abgas-/Luft-Verdünnungsverhältnisses und mit einer Abgasaufnahmeleitung (2), der die Abgase eines zu testenden Motors zuführbar sind, wobei die Teilstromverdünnungseinrichtung folgende Merkmale aufweist:

   - eine Abgasentnahmesonde (4), mit der ein Abgasteilstrom $\dot{m}_{SON}$ aus der Abgasaufnahmeleitung (2) entnehmbar und über eine diesen messende Massenstromsonde (5) sowie eine anschließende Abgaseinspeiseleitung (6) in einen Mischkanal (7) einspeisbar ist, der einenendes an einen eingangs eine Einlaufdüse (8) mit Wirkdruckmeßeinrichtung aufweisenden Verdünnungsluftansaugkanal (10) und anderenendes über einen Absaugkanal (11) an ein Sauggebläse (12) angeschlossen ist und aus dessen Mischzone luftverdünnte Abgase für Analysezwecke entnehmbar sind,

   - je eine Drosselklappe (K1, K2) vor bzw. hinter dem Mischkanal (7) in den dortigen Leitungswegen (10, 11) zur Einstellung der jeweiligen Massenströme und Drücke,

   - je eine Drosselklappe (K3, K4) strömungsmäßig vor bzw. hinter der Abgasentnahmestelle (4/1) in der Abgasaufnahmeleitung (2) für eine einen störungsfreien Betrieb des zu testenden Motors bewirkende Abgasgegendruckeinstellung und eine Abgasdruckvariierung im Abgasentnahmebereich, sowie

   - Regel- und Steuereinrichtungen zur bedarfsgerechten Einstellung des Abgas-/Luft-Verdünnungsverhältnisses

$$q = \frac{\dot{m}_{DIL} + \dot{m}_{SON}}{\dot{m}_{SON}}$$

   durch entsprechende Verstellung der Drosselklappen (K1, K2, K3, K4) anhand erfaßter Massenstromwerte $\dot{m}_{DIL}$, Verdünnungsluftstrom,

gemessen an Einlaufdüse (8), $\dot{m}_{SON}$, Abgasteilstrom, gemessen mit Massenstromsonde (5).

2. Anlage nach Anspruch 1, gekennzeichnet durch eine Meßwerterfassung, Meßwertverarbeitung und zumindest teilweiwse Regelung und Steuerung des Abgas-/Luft-Verdünnungsprozesses, insbesondere der Drosselklappenverstellung mittels eines Computersystems (PC), das hard- uns softwaremäßig zweckentsprechend ausgestattet ist.

3. Anlage nach einem der vorhergehenden Ansprüche, dadurch gekennzeichnet, daß zusätzlich zu den Einrichtungen, die die Bestimmung des Abgas-/Luft-Verdünnungsverhältnisses nach der Massenbilanz-Methode ermöglichen, Einrichtungen zur Messung des $CO_2$-Gehaltes des Rohabgases (22), des verdünnten Abgases (23) und der Verdünnungsluft sowie ein $CO_2$-Analysator (24) vorgesehen sind, mit dem auch eine Bestimmung des Abgas-/Luft-Verdünnungsverhältnisses nach der $CO_2$-Bilanz-Methode durchführbar ist, deren Ergebnisse zu Vergleichszwecken mit den Ergebnissen der MassenbilanzMethode heranziehbar sind.

4. Anlage nach Anspruch 1, dadurch gekennzeichnet, daß das rohrförmige Gasaufnahmeteil (4/1) der Abgasentnahmesonde (4) mittig, insbesondere koaxial in der Abgasaufnahmeleitung (2) verlaufend im Rohabgasstrom angeordnet und anschließend nach außen zur Massenstromsonde (5) hingeführt ist.

5. Anlage nach Anspruch 4, dadurch gekennzeichnet, daß der Abgasaufnahmeteil (4/1) der Abgasentnahmesonde (4) räumlich kurz vor der Drosselklappe (K4) angeordnet ist.

6. Anlage nach Anspruch 1, dadurch gekennzeichnet, daß die Abgaseinspeiseleitung (6) mit ihrem Mündungsteil (6/1) mittig, insbesondere konzentrisch im Mischkanal (7) angeordnet ist und dort mit ihrer Austrittsöffnung vor oder im Bereich einer eine querschnittsgrößere Durchlaßöffnung (21/1) aufweisenden Lochblende (21) endet, nach der die Abgas-/Luftmischstrecke beginnt.

7. Anlage nach Anspruch 6, dadurch gekennzeichnet, daß die Abgas-/Luftmischstrecke bei einem Mischkanaldurchmesser von ca. 150 mm eine Länge von etwa 1450 mm, die Durchlaßöffnung (21/1) der Lochblende (21) einen Durchmesser von etwa 100 mm und der Mündungsteil (6/1) der Abgaseinspeiseleitung (6) einen Durchmesser von etwa 25 mm hat, wohingegen der Abgasaufnahmeteil (4/1) der Abgasentnahmesonde (4) bei einem Innendurchmesser der Abgasaufnahmeleitung (2) von ca. 100 mm nur einen Durchmesser von etwa 12 mm hat.

**8.** Anlage nach Anspruch 1, dadurch gekennzeichnet, daß das einzig notwendige Gebläse (12) ein mit konstanter Drehzahl laufendes Sauggebläse ist.

**9.** Anlage nach Anspruch 1, dadurch gekennzeichnet, daß der Verdünnungsluftansaugkanal (10) nach der Einlaufdüse (8) einen etwa 2/3 jenes des Mischkanals (7) betragenden Durchmesser hat und im Bereich nach der Drosselklappe (K1) kontinuierlich auf den Durchmesser des Mischkanals (7) erweitert ist.

**10.** Anlage nach Anspruch 9, dadurch gekennzeichnet, daß die Verdünnungsluft ungefiltert und ohne über einen Wärmetauscher geführt zu werden durch den Verdünnungsluftansaugkanal (10) in den Mischkanal (7) eingespeist wird.

**11.** Anlage nach Anspruch 9, dadurch gekennzeichnet, daß die Einlaufdüse (8) durch ein Venturirohrstück gebildet ist, das mit seinem verengten Trichterquerschnitt am Ende des Verdünnungsluftkanals (10) austauschbar angeschlossen ist.

**12.** Anlage nach Anspruch 11, dadurch gekennzeichnet, daß zum Analysieren der Abgase unterschiedlich hubraumgroßer Motoren entsprechend angepaßte unterschiedlich groß kalibrierte Einlaufdüsen (8) vorgesehen sind und die jeweils notwendige Einlaufdüse (8) am eintrittsseitigen freien Ende des Verdünnungsluftansaugkanals (10) befestigt wird.

**13.** Anlage nach einem oder mehreren der vorhergehenden Ansprüche, dadurch gekennzeichnet, daß die Einlaufdüse (8) mit einem Druckwandler kombiniert ist, der den Wirkdruck der angesaugten Verdünnungsluft erfaßt, kleine Über-, Unter- und Differenzdrücke feststellt und ein durckproportionales Spannungssignal liefert sowie die erfaßten Werte auf einer Anzeigeeinrichtung, insbesondere LCD-Display optisch zur Anzeige bringt.

**14.** Anlage nach Anspruch 1, dadurch gekennzeichnet, daß mittels der Massenstromsonde (5) der Abgasteilstrom $\dot{m}_{SON}$ über eine Durchflußmessung nach dem Staudruckprinzip bestimmt wird.

**15.** Anlage nach Anspruch 1, dadurch gekennzeichnet, daß sie als mobile Einheit konzipiert ist und hierzu alle ihre Teile auf bzw. an einer mit Rädern (25) versehenen Plattform (26) angeordnet sind.

**Claims**

**1.** System for pollutant analysis, especially particle emission, of diesel engine exhaust gas, having a sampling flow dilution device for adjusting and determining the exhaust gas/air dilution ratio and having an exhaust gas receiver line (2), to which the exhaust gases from an engine to be tested can be fed, the sampling flow dilution device having the following characteristic features:

- an exhaust gas sampling probe (4), by means of which an exhaust gas sampling flow $\dot{m}_{SON}$ can be drawn from the exhaust gas receiver line (2) and fed by way of a flow-measuring mass flow probe (5) and a connecting exhaust gas feed line (6) into a mixing duct (7), which is connected at one end to a dilution air intake duct (10) having an inlet nozzle (8) at the entrance thereof with an effective pressure measuring device, and at the other end by way of an extraction duct (11) leading to a suction fan (12) and from the mixing zone of which duct air-diluted exhaust gases can be drawn off for purposes of analysis,

- a throttle valve (K1, K2) in each of the lines (10, 11) respectively upstream and downstream of the mixing duct (7) for adjusting the respective mass flows and pressures,

- a throttle valve (K3, K4) upstream and downstream respectively of the exhaust gas sampling point (4/1) in the exhaust gas receiver line (2) for adjustment of the exhaust gas back pressure producing smooth operation of the engine to be tested and for varying the exhaust gas pressure in the exhaust gas sampling area, and

- regulating and control devices for adjustment of the exhaust gas/air dilution ratio

$$q = \frac{\dot{m}_{DIL} + \dot{m}_{SON}}{\dot{m}_{SON}}$$

as required, by corresponding adjustment of the throttle valves (K1, K2, K3, K4) on the basis of registered mass flow values $\dot{m}_{DIL}$, dilution air flow, measured at the inlet nozzle (8), $\dot{m}_{SON}$ exhaust gas sampling flow, measured by the mass flow probe (5).

**2.** System as claimed in claim 1, characterised by data acquisition, data processing and at least partial regulation and control of the exhaust gas/air dilution process, in particular of the throttle valve adjustment by means of a computer system (PC), which is equipped with hardware and software suitable for the purpose.

**3.** System as claimed in one of the preceding claims, characterised in that, in addition to the devices allowing the exhaust gas/air dilution ratio to be determined by the mass balance method, devices are provided for measuring the $CO_2$ content of the untreated exhaust gas (22), the diluted exhaust gas (23) and the dilution air together with a $CO_2$ analyser (24), by means of which determination of the exhaust gas/air dilution ratio can also be performed by the $CO_2$ balance method, the results of which can be used for purposes of comparison with the results of the mass balance method.

4. System as claimed in claim 1, characterised in that the tubular gas receiver section (4/1) of the exhaust gas sampling probe (4) is arranged in the untreated exhaust gas flow running centrally, especially coaxially in the exhaust gas receiver line (2) and is then led outwards to the mass flow probe (5).

5. System as claimed in claim 4, characterised in that the exhaust gas receiver section (4/1) of the exhaust gas sampling probe (4) is spatially arranged immediately upstream of the throttle valve (K4).

6. System as claimed in claim 1, characterised in that the exhaust gas feed line (6) is arranged with its outlet section (6/1) centrally, especially concentrically in the mixing duct (7), where it terminates with its outlet orifice upstream or in the area of an apertured diaphragm (21) having a passage opening (21/1) of larger cross-section, downstream of which the exhaust gas/air mixing line begins.

7. System as claimed in claim 6, characterised in that the exhaust gas/air mixing line with a mixing duct diameter of approx 150mm has a length of approximately 1450mm, the passage opening (21/1) of the apertured diaphragm (21) has a diameter of approximately 100mm and the outlet section (6/1) of the exhaust gas feed line (6) has a diameter of approximately 25mm, whereas the exhaust gas receiver section (4/1) of the exhaust gas sampling probe (4) only has a diameter of approximately 12mm for an inside diameter of the exhaust gas receiver line (2) of approximately 100mm.

8. System as claimed in claim 1, characterised in that the sole fan (12) required is a suction fan running at constant rotational speed.

9. System as claimed in claim 1, characterised in that the dilution air intake duct (10) downstream of the inlet nozzle (8) has a diameter approximately 2/3 that of the mixing duct (7) and in the area downstream of the throttle valve (K1) continuously widens to the diameter of the mixing duct (7).

10. System as claimed in claim 9, characterised in that the dilution air is fed through the dilution air intake duct (10) into the mixing duct (7) unfiltered and without being led through a heat exchanger.

11. System as claimed in claim 9, characterised in that the inlet nozzle (8) is formed by a venturi tube section, which is replaceably connected by its narrowed funnel cross-section to the end of the dilution air duct (10).

12. System as claimed in claim 11, characterised in that, for analysing the exhaust gases from engines of differing displacement, correspondingly adapted inlet nozzles (8) of differing calibrated size are provided and that the inlet nozzle (8) required in each case is fixed to the inlet-side free end of the dilution air intake duct (10).

13. System as claimed in one or more of the preceding claims, characterised in that the inlet nozzle (8) is combined with a pressure transducer which senses the effective pressure of the intake dilution air, detects slight excess, negative and differential pressures and delivers a voltage signal proportional to the pressure and displays the values registered visually on a display device, especially an LCD.

14. System as claimed in claim 1, characterised in that the exhaust gas sampling flow $\dot{m}_{SON}$ is determined by means of the mass flow probe (5) by a flow measurement on the dynamic pressure principle.

15. System as claimed in claim 1, characterised in that it is designed as a mobile unit and that for this purpose all its parts are arranged on or against a platform (26) provided with wheels (25).

**Revendications**

1. Installation pour l'analyse des matières polluantes notamment d'émission de particules des gaz d'échappement de moteur diesel, comprenant une installation de dilution à courant partiel pour régler et définir le rapport de dilution gaz d'échappement/air et un collecteur de gaz d'échappement (2) qui reçoit les gaz d'échappement d'un moteur à contrôler, l'installation de dilution à courant partiel comprenant les caractéristiques suivantes :

   - une sonde de prélèvement de gaz d'échappement (4) et qui prélève un courant partiel de gaz d'échappement $\dot{m}_{SON}$ du collecteur de gaz d'échappement (2) et fournit ce gaz à un canal de mélange (7) par l'intermédiaire d'une sonde massique (5) mesurant le gaz suivie d'une conduite d'injection de gaz d'échappement (6), ce canal de mélange étant relié par une extrémité à un canal d'aspiration d'air de dilution (10) dont l'entrée est munie d'une buse d'entrée (8) d'une installation de mesure de pression active et dont l'autre extrémité est reliée à une machine aspirante (12) par l'intermédiaire d'un canal d'aspiration (11), et cette zone de mélange fournissant des gaz d'échappement dilués par de l'air pour des fins d'analyse,

- chaque fois un volet d'étranglement (K1, K2) en amont ou en aval du canal de mélange (7) dans les branches des conduites (10, 11) correspondantes pour régler les débits massiques et de pression respectifs,
- un volet d'étranglement (K3, K4) en amont ou en aval du point de prélèvement des gaz d'échappement (4/1) dans le collecteur de gaz d'échappement (2) pour un fonctionnement sans incident du réglage de pression antagoniste des gaz d'échappement pour le moteur à tester et une variation de la pression des gaz d'échappement dans la plage de prélèvement des gaz d'échappement, ainsi que
- une installation de régulation et de commande pour régler de façon appropriée le rapport de dilution gaz d'échappement/air

$$q = \frac{\dot{m}_{DIL} + \dot{m}_{SON}}{\dot{m}_{SON}},$$

par un réglage correspondant des volets d'étranglement (K1, K2, K3, K4) à l'aide des valeurs détectées du débit massique $\dot{m}_{DIL}$/courant d'air de dilution, mesuré sur la buse d'entrée (8), $\dot{m}_{SON}$/débit partiel de gaz d'échappement mesuré par la sonde de débit massique (5).

2. Installation selon la revendication 1, caractérisée par une détection de valeurs de mesure, un traitement des valeurs de mesure et au moins une régulation et une commande partielle du procédé de dilution gaz d'échappement/air, notamment le réglage des papillons d'étranglement se fait à l'aide d'un système d'ordinateur (PC) conçu et programmé de manière appropriée.

3. Installation selon l'une des revendications précédentes, caractérisée en ce qu'en plus des installations permettant de déterminer le rapport de dilution gaz d'échappement/air selon le procédé du bilan massique, elle comporte des installations pour mesurer la teneur en $CO_2$ des gaz d'échappement bruts (22), les gaz d'échappement dilués (23) et de l'air de dilution ainsi qu'un analyseur (24) de $CO_2$, qui permet également de déterminer le rapport de dilution gaz d'échappement/air selon le procédé du bilan de $CO_2$ dont les résultats sont utilisés pour permettre la comparaison avec les résultats du procédé du bilan massique.

4. Installation selon la revendication 1, caractérisée en ce que la partie de réception de gaz (4/1), tubulaire de la sonde de prélèvement de gaz (4) est disposée au milieu notamment de manière coaxiale dans le collecteur de gaz d'échappement (2), dans le courant de gaz d'échappement brut pour sortir du collecteur et rejoindre la sonde de débit massique (5).

5. Installation selon la revendication 4, caractérisée en ce que la partie de réception de gaz d'échappement (4/1) de la sonde de prélèvement de gaz d'échappement (4) se trouve directement en amont du volet d'étranglement (K4).

6. Installation selon la revendication 1, caractérisée en ce que la conduite d'injection de gaz d'échappement (6) est placée avec sa partie d'embouchure (6/1), au milieu notamment en position concentrique dans le canal de mélange (7) et se termine à cet endroit par son orifice de sortie en avant ou au niveau d'un diaphragme (21) dont l'orifice (21/1) a une section plus grande, et en aval de ce diaphragme commence le chemin de mélange des gaz d'échappement et de l'air.

7. Installation selon la revendication 6, caractérisée en ce que le chemin de mélange gaz d'échappement/air pour un canal de mélange d'un diamètre environ de 150 mm a une longueur de 1450 mm, l'orifice (21/1) du diaphragme (21) a un diamètre environ de 100 mm et la partie d'embouchure (6/1) de la conduite d'injection de gaz d'échappement (6) a un diamètre environ de 25 mm, la partie de réception (4/1) des gaz d'échappement de la sonde de prélèvement de gaz d'échappement (4) ayant seulement un diamètre environ de 12 mm pour une conduite de prélèvement de gaz d'échappement (2) dont le diamètre intérieur est environ de l'ordre de 100 mm.

8. Installation selon la revendication 1, caractérisée en ce que la seule machine (12) nécessaire est une machine aspirante à vitesse de rotation constante.

9. Installation selon la revendication 1, caractérisée en ce que le canal d'aspiration d'air de dilution (10) en aval de la buse d'entrée (8) a un diamètre correspondant sensiblement à 2/3 de celui du canal de mélange (7) et va en s'élargissant en continu pour rejoindre le diamètre du canal de mélange (7) en aval du volet d'étranglement (K1).

10. Installation selon la revendication 9, caractérisée en ce que l'air de dilution est fourni non filtré et sans traverser l'échangeur de chaleur au canal d'aspiration d'air de dilution (10) pour être introduit dans le canal de mélange (7).

11. Installation selon la revendication 9, caractérisée en ce que la buse d'entrée (8) est conçue sur le principe d'un tube venturi dont la section d'entonnoir, réduite est reliée de manière interchangeable à l'extrémité du canal de dilution d'air (10).

**12.** Installation selon la revendication 11, caractérisée en ce que pour analyser les gaz d'échappement de moteurs à cylindrées différentes on prévoit des buses d'entrée (5) de calibre différent et la buse d'entrée (8) nécessaire à chaque fois est fixée à l'extrémité libre du côté de l'entrée du canal d'aspiration d'air de dilution (10).

**13.** Installation selon une ou plusieurs des revendications précédentes, caractérisée en ce que la buse d'entrée (8) est combinée à un convertisseur de pression qui détecte la pression active de l'air de dilution aspiré, constate de faibles surpressions ou dépressions et les différences de pression et fournit un signal de tension proportionnel à la pression ainsi que les valeurs détectées à une installation d'affichage notamment un affichage à cristaux liquides (affichage LCD) pour l'affichage optique.

**14.** Installation selon la revendication 1, caractérisée en ce que le courant partiel des gaz d'échappement $\dot{m}_{SON}$ est défini à l'aide de la sonde de débit massique (5) par une mesure de débit selon le principe de la pression dynamique.

**15.** Installation selon la revendication 1, caractérisée en ce qu'elle est conçue comme ensemble mobile et que toutes ses pièces sont montées sur une plate-forme (26) équipée de roues.

FIG. 1

FIG.2

FIG.3

FIG.4

EP 0 471 174 B1